# EUROPEAN PATENT APPLICATION

(11) **EP 4 585 084 A1**
(43) Date of publication of application: **16.07.2025**
(21) Application number: 23864465.2
(22) Date of filing: 05.07.2023
(51) Int. Cl.: A24F 40/57

(54) **AEROSOL GENERATION DEVICE, CONTROL METHOD THEREFOR, AND CONTROL DEVICE**

(30) Priority: 15.09.2022 CN 202211122729
(71) Applicant: Shenzhen Merit Technology Co., Ltd., Shenzhen, Guangdong 518105 (CN)
(72) Inventor: GUO, Yu, Shenzhen, Guangdong 518105 (CN); XIAN, Xiaoyi, Shenzhen, Guangdong 518105 (CN); GUO, Fengwen, Shenzhen, Guangdong 518105 (CN); LIANG, Feng, Shenzhen, Guangdong 518105 (CN); DU, Xianwu, Shenzhen, Guangdong 518105 (CN); LIU, Xiaoli, Shenzhen, Guangdong 518105 (CN)
(74) Representative: Manitz Finsterwald Patent- und Rechtsanwaltspartnerschaft mbB
(86) International application number: PCT/CN2023/105858
(87) International publication number: WO 2024/055728

(57) **Abstract**

An aerosol generation device, a control method therefor, and a control device. The aerosol generation device comprises a power supply (150), a processor (110), a heating device (120), and a vaping detection sensor (130). The power supply (150) is used for supplying energy to the heating device (120). The processor (110) is used for, after detecting a starting instruction, controlling a supply of energy to the heating device (120) to cause the heating device to reach a first temperature, the first temperature being less than or equal to the lowest temperature for generating aerosol. The processor (110) is further used for acquiring detection data of the vaping detection sensor (130), determining whether there is a vaping action according to the detection data, and stabilizing the heating device (120) at the first temperature if not detecting the vaping action. Thus, the power consumption of the aerosol generation device is reduced, saving energy.

## Description

### CROSS-REFERENCE TO RELATED APPLICATION

This application claims priority to Chinese Patent Application No. 202211122729.7, filed with the China National Intellectual Property Administration on September 15, 2022, and entitled "Aerosol Generation Device, Control Method therefor, and Control Device", which is incorporated herein by reference in its entirety.

### TECHNICAL FIELD

The present invention relates to the field of atomization technology, and particularly to an aerosol generation device, a control method and a control apparatus thereof.

### BACKGROUND

The aerosol generation device is a kind of atomization device, which can be used for heating an aerosol generating substrate to generate an aerosol. The aerosol generating substrate may be a solid substrate formed by plant stems and leaves, etc., or a liquid substrate such as tobacco oil. When a solid substrate is heated, since the preheating speed is low, in order to facilitate the inhalation by the user and ensure the continuous generation of the aerosol, a continuous heating mode is generally adopted, but such a mode consumes a lot of power.

### SUMMARY

In view of this, in order to address the above technical problem, it is necessary to provide an aerosol generation device, a control method and a control apparatus thereof capable of reducing power consumption.

In the first aspect of the present application, an aerosol generation device is provided, including a power supply, a processor, a heating component, and an inhalation detection sensor;
the power source is configured to supply energy to the heating component;
the processor is configured to control the energy supplied to the heating component after detecting a startup instruction, to allow the heating component to reach a first temperature which is less than or equal to a minimum temperature for generating an aerosol;
the processor is further configured to acquire detection data of the inhalation detection sensor, determine whether there exists an inhalation action according to the detection data, and control the heating component to stabilize at the first temperature when no inhalation action is detected.

In an embodiment, the processor is further configured to control the heating component to reach a second temperature from the first temperature when the inhalation action is detected, wherein the second temperature is greater than the first temperature.

In an embodiment, the processor is further configured to control the heating component to cool down from the second temperature to the first temperature after the inhalation action is completed.

In an embodiment, the processor is further configured to: control the heating component to heat up to a third temperature after detecting the startup instruction, the third temperature being greater than the first temperature; and control the heating component to cool down from the third temperature to the first temperature.

In an embodiment, the heating component is configured to receive an aerosol generating substrate and heat around the aerosol generating substrate, the first temperature is less than 200°C, the second temperature is greater than 200°C, and the third temperature is greater than 200°C.

In an embodiment, the first temperature is between 170°C and 180°C, the second temperature is between 210°C and 240°C, and the third temperature is between 240°C and 260°C.

In an embodiment, the heating component is configured to be inserted into the aerosol generating substrate and heat the aerosol generating substrate from an inside of the aerosol generating substrate, the first temperature is less than 300°C, the second temperature is greater than 300°C, and the third temperature is greater than 300°C.

In an embodiment, the aerosol generation device further includes a temperature measuring element, the processor is further configured to:
acquire a detected temperature of the heating component through the temperature measuring element;
perform a PID calculation according to the detected temperature and a target temperature to obtain a target energy, and control the heating component to reach a corresponding target temperature according to the target energy; the target temperature includes the first temperature, the second temperature or the third temperature.

In an embodiment, the processor is further configured to:
acquire an energy supplied to the heating component, and stop supplying the energy to the heating component when the energy supplied to the heating component exceeds preset energy.

In the second aspect of the present application, a control method for an aerosol generation device is provided, including:
controlling a heating component in the aerosol generation device to reach a first temperature after a startup instruction is detected, the first temperature being less than or equal to a minimum temperature for generating an aerosol;
acquiring detection data of an inhalation detection sensor in the aerosol generation device, and determining whether there exists an inhalation action according to the detection data;
controlling the heating component to stabilize at the first temperature when no inhalation action is detected.

In an embodiment, the method further includes:
after acquiring the detection data of the inhalation detection sensor in the aerosol generation device and determining whether there exists the inhalation action according to the detection data,
controlling the heating component to reach the second temperature from the first temperature when the inhalation action is detected, the second temperature being greater than the first temperature.

In an embodiment, the method further includes:
after controlling the heating component to reach the second temperature from the first temperature when the inhalation action is detected,
controlling the heating component to cool down from the second temperature to the first temperature when the inhalation action is completed.

In an embodiment, the method further includes:
after controlling the heating component in the aerosol generation device to reach the first temperature after the startup instruction is detected,
controlling the heating component in the aerosol generation device to heat up to the third temperature after the startup instruction is detected; and
controlling the heating component to cool down from the third temperature to the first temperature.

In the third aspect of the present application, a control apparatus for an aerosol generation device is provided, including:
a first control module, configured to control a heating component in the aerosol generation device to reach a first temperature after a startup instruction is detected, wherein the first temperature is less than or equal to a minimum temperature for generating an aerosol;
an inhalation determination module, configured to acquire detection data of an inhalation detection sensor in the aerosol generation device, and determine whether there exists an inhalation action according to the detection data;
the first control module is further configured to control the heating component to stabilize at the first temperature when no inhalation action is detected.

According to the above-mentioned aerosol generation device, control method and control apparatus thereof, after the startup instruction is detected, the heating component in the aerosol generation device is controlled to reach the first temperature, the first temperature is less than or equal to the minimum temperature for generating the aerosol, so that the heating component can preheat the aerosol generating substrate; and then the detection data of the inhalation detection sensor in the aerosol generation device is acquired, and whether there exists an inhalation action is determined according to the detection data; when no inhalation action is detected, the heating component is controlled to stabilize at the first temperature. In such a manner, after the aerosol generation device is turned on, when no inhalation action is detected, the heating component is controlled to stabilize at the first temperature which is lower than the minimum temperature for generating the aerosol. Compared to the conventional technology (continuous heating after being turned on), a lot of energy can be saved and the power consumption can be reduced without affecting the user use.

### BRIEF DESCRIPTION OF THE DRAWINGS

FIG. 1 is a schematic structure diagram of an aerosol generation device according to an embodiment of the present invention.
FIG. 2 is a schematic relationship diagram of a temperature of a heating component over time according to an embodiment of the present invention.
FIG. 3 is a schematic relationship diagram of a temperature of a heating component over time according to another embodiment of the present invention.
FIG. 4 is a schematic structure diagram of an aerosol generation device according to another embodiment of the present invention.
FIG. 5 is a schematic structure diagram of an aerosol generation device according to another embodiment of the present invention.
FIG. 6 is a schematic relationship diagram of a temperature of a heating component over time according to another embodiment of the present invention.
FIG. 7 is a flow chart showing a control method for an aerosol generation device according to an embodiment of the present invention.
FIG. 8 is a schematic diagram of a detailed process for controlling a heating component in an aerosol generation device to reach a first temperature after detecting a startup instruction according to an embodiment of the present invention.
FIG. 9 is a schematic diagram of a module structure of a control apparatus for an aerosol generation device according to an embodiment of the present invention.

### DETAILED DESCRIPTION OF THE EMBODIMENTS

In order to make the purpose, technical solution and advantages of the present application more clearly understood, the present application is further described in detail below in conjunction with the accompanying drawings and embodiments. It should be understood that the specific embodiments described herein are merely used for illustrating the present application, rather than limiting the present application.

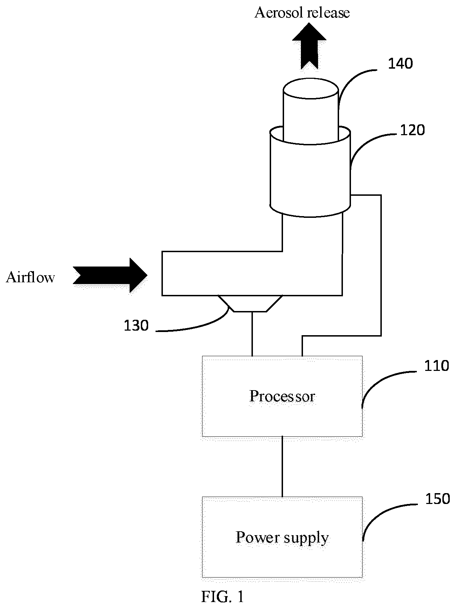

In an embodiment, as shown in FIG. 1, an aerosol generation device is provided. The aerosol generation device may include: a processor 110, a heating component 120, an inhalation detection sensor 130, and a power source 150. The power source 150 is configured to supply energy to the heating component 120, and the power source 150 may be a battery, such as a rechargeable lithium-ion battery, a nickel-metal hydride battery, a nickel-cadmium battery or a lithium-based battery. The heating component 120 is also referred to as a heater, which may have various forms, such as a heating sheet, a heating needle, a heating rod, a heating wire or thread, a heating tube, etc. Alternatively, the heating component 120 may also be a combination of two or more different forms of heating components. In the embodiment, the heating component 120 is a circumferential-shape heating body and has a groove or a hole for inserting the aerosol generating substrate 140. The heating component wraps and heats around the aerosol generating substrate 140 to generate the aerosol.

The heating mode of the heating component 120 may be infrared radiation heating, that is, a material with a higher infrared emissivity serves as a heating substrate or a heating coating to heat the aerosol generating substrate. Since infrared radiation heating heats up quickly, it can achieve rapid temperature increase when inhalation is detected. Of course, in a specific implementation, the heating component may also adopt other heating modes, such as electromagnetic heating, laser heating, microwave heating, electric field heating, etc., as long as it can heat up quickly.

The inhalation detection sensor 130 may be a semiconductor air pressure sensor, a flow sensor, or the like, which detects an inhalation action by detecting an air pressure change in an airflow channel during the inhalation. Alternatively, the inhalation detection sensor 130 may also be a sensor that detects the inhalation action by detecting a temperature change in the airflow channel during the inhalation. For example, a thermocouple that converts a temperature difference into a potential difference, and a thermistor that converts a temperature change into a resistance change, etc. The inhalation detection sensor 130 is provided at a side of the airflow channel or inside the airflow channel where the airflow is generated by inhalation.

The processor 110 is connected to the heating component 120, the inhalation detection sensor 130, and the power source 150. The processor 110 is configured to obtain sensor data (including data of the inhalation detection sensor 130) to control an operating power of the heating component 120. The aerosol generation device shown in FIG. 1 is an example. In a specific implementation, the technical solution of the present application can be applied to any aerosol generation device.

Specifically, the processor 110 is configured to control the power supply 150 to supply the energy to the heating component 120 after detecting the startup instruction, to allow the heating component 120 to reach a first temperature which is less than or equal to the minimum temperature for generating the aerosol. The processor 110 is further configured to acquire detection data of the inhalation detection sensor 130, determine whether there exists an inhalation action according to the detection data, and control the heating component 120 to stabilize at the first temperature when no inhalation action is detected.

Specifically, the processor in the aerosol generation device can detect whether a startup instruction is received, in which the startup instruction can be generated by a user operating a power-on instruction on the aerosol generation device. As an example, the aerosol generation device may further include a button for generating a power-on instruction, and the user may press or touch the button to generate a corresponding power-on instruction. Alternatively, there may exist an acceleration sensor on the aerosol generation device. In user's use, the acceleration sensor may generate an acceleration, so that the aerosol generation device generates a corresponding startup instruction.

After the startup instruction is detected, the processor in the aerosol generation device controls the heating component in the aerosol generation device to reach the first temperature, in which the first temperature is less than or equal to the minimum temperature for generating the aerosol. Specifically, the processor may control the energy provided by the power supply to the heating component to allow the heating component to reach the first temperature.

Furthermore, in order to improve the user experience, the heating component needs to reach the first temperature as quickly as possible to preheat the aerosol generating substrate in the aerosol generation device, so that the user can smoke the aerosol in less time without having to wait for a long time. In the embodiment, the time required to control the heating component to reach the first temperature is less than 10 seconds.

The aerosol generation device acquires the detection data of the inhalation detection sensor in the aerosol generation device, and determines whether there exists an inhalation action according to the acquired detection data. The detection data may be air pressure change data of the airflow channel, or temperature change data of the airflow channel.

It should be appreciated that the aerosol generation device may also detect the inhalation action by detecting the temperature change of the heating component through a temperature measuring element. In this case, the temperature measuring element is provided in the airflow channel of the aerosol generation device as an inhalation detection sensor.

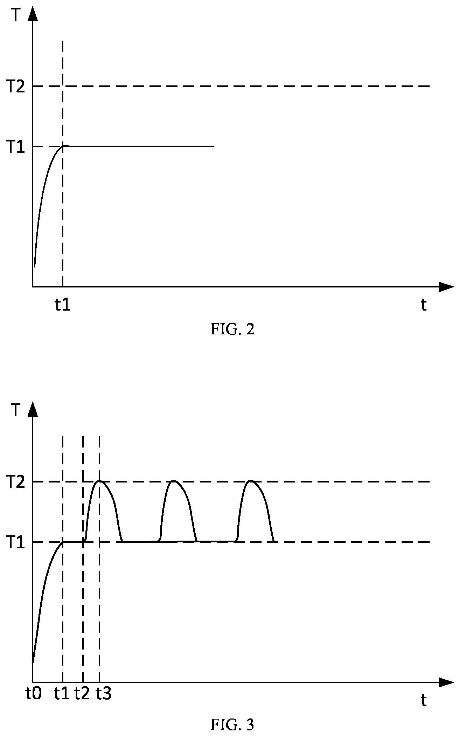

If no inhalation action is detected, the heating component 120 is controlled to stabilize at the first temperature which is less than or equal to the minimum temperature for generating the aerosol. As shown in FIG. 2, T1 represents the first temperature.

It should be appreciated that, for ease of use, the first temperature should not be much lower than the minimum temperature for generating the aerosol. As an example, the first temperature is less than 200°C, a second temperature is greater than 200°C, and a third temperature is greater than 200°C. Specifically, the first temperature is between 170°C and 180°C, that is, the first temperature can be any temperature value between 170°C and 180°C. In this case, the minimum temperature for generating the aerosol is 180°C. The first temperature should not be set to 100°C, because when the user uses it, the aerosol generation device may need more time to heat the heating component to the required temperature than when the first temperature is equal to 180°C. In other embodiments, the heating component is inserted into the aerosol generating substrate to heat the aerosol generating substrate from the inside to generate the aerosol, in this case, the first temperature is less than 300°C, the second temperature is greater than 300°C, and the third temperature is greater than 300°C.

When an inhalation action is detected, the heating component is controlled to reach the second temperature from the first temperature, in which the second temperature is greater than the first temperature and is greater than or equal to a minimum temperature for generating the aerosol, that is, the heating component is controlled to heat to atomize the aerosol generating substrate to form the aerosol.

After the inhalation action is determined to be completed according to the detection data of the inhalation detection sensor, the processor in the aerosol generation device controls the heating component to cool down from the second temperature to the first temperature.

As an example, as shown in FIG. 3, at the moment t0, a startup instruction is detected, and the processor in the aerosol generation device controls the heating component in the aerosol generation device to reach the first temperature T1 within time t1; if no inhalation action is detected, the heating component is controlled to stabilize at the first temperature T1. At the moment t2, the inhalation action is detected, and the heating component is controlled to increase the temperature from the first temperature T1 to the second temperature T2, so that the aerosol generating substrate is atomized to form the aerosol. At the moment t3, it is determined that the inhalation action stops according to the detection data of the inhalation detection sensor, and the processor controls the power supply to reduce the energy supplied to the heating component, so that the temperature of the heating component gradually decreases to the first temperature T1 and is maintained at the first temperature T1. When the next inhalation action is detected, the operations from moment t2 to moment t3 are repeated, and the cycle is repeated. If a power-off instruction is detected, the power supply stops supplying power to the processor and other modules, the aerosol generation device is in an off state, and the temperature of the heating component decreases to the normal temperature. The normal temperature refers to an ambient temperature of the environment where the aerosol generation device is located.

As an example, when the heating component is a circumferential-shape heating body, the first temperature is less than 200°C, the second temperature is greater than 200°C, and the third temperature is greater than 200°C. Specifically, the second temperature may be between 210°C and 240°C, that is, the second temperature may be any temperature value between 210°C and 240°C.

The above-mentioned aerosol generation device includes the processor, the heating component and the inhalation detection sensor. After detecting the startup instruction, the processor controls the heating component in the aerosol generation device to reach the first temperature which is less than or equal to the minimum temperature for generating the aerosol, so that the heating component can preheat the aerosol generating substrate; then the processor acquires the detection data of the inhalation detection sensor in the aerosol generation device, determines whether there exists an inhalation action according to the detection data, and controls the heating component to stabilize at the first temperature when no inhalation action is detected. In such a manner, after the aerosol generation device is turned on, when no inhalation action is detected, the heating component is controlled to stabilize at the first temperature which is lower than the minimum temperature for generating the aerosol. Compared to the conventional technology (i.e., continuous heating after being turned on), the aerosol generation device of the present application can save a lot of energy and reduce power consumption without affecting user use.

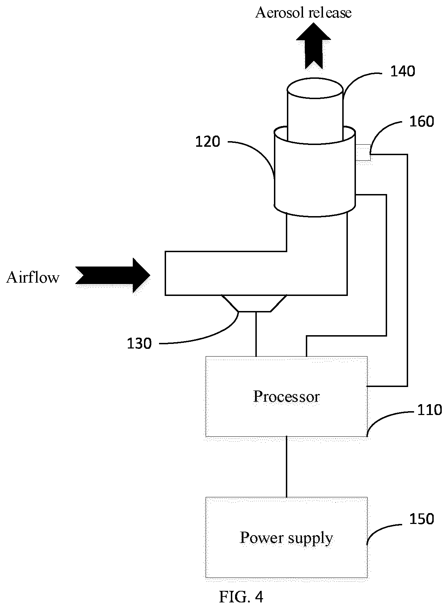

In an embodiment, as shown in FIG. 4, an aerosol generation device is provided. The aerosol generation device may include: a processor 110, a heating component 120, an inhalation detection sensor 130, a power supply 150, and a temperature measuring element 160. The processor 110 is connected to the heating component 120, the inhalation detection sensor 130, the power supply 150, and the temperature measuring element 160 respectively. The inhalation detection sensor 130 is configured to detect an inhalation action according to a pressure change or temperature change in the airflow channel. The temperature measuring element 160 is configured to detect a temperature of the heating component 120. The embodiment shown in FIG. 2 differs from the embodiment shown in FIG. 1 in that the temperature measuring element 160 is added in the embodiment, and other components are the same as those in the embodiment shown in FIG. 1. The processor 110 is configured to acquire data of at least one of the inhalation detection sensor 130 and the temperature measuring element 160 to control an operating power of the heating component 120. Specifically, the processor 110 determines whether there exists an inhalation action according to the data of the inhalation detection sensor 130, and the processor 110 acquires the temperature of the heating component 120 according to the data of the temperature measuring element 160.

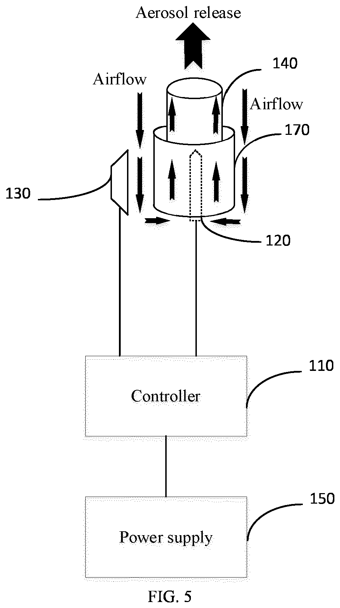

In an embodiment, as shown in FIG. 5, an aerosol generation device is provided. The aerosol generation device may include: a processor 110, a heating component 120, an inhalation detection sensor 130, a power source 150, and a fixing support 170. The heating component 120 is an inserted heating body (indicated by a dashed line shape in FIG. 5). The heating body is inserted into the aerosol generating substrate 140 to heat the aerosol generating substrate 140 from the inside. The fixing support 170 is configured to fix the aerosol generating substrate 140. When the heating component 120 is an inserted-type heating body, the first temperature is less than 300°C, the second temperature is greater than 300°C, and the third temperature is greater than 300°C. It should be noted that, compared to the circumferential-shape heating component, the first temperature, the second temperature and the third temperature of the inserted-type heating component are higher. This is because the inserted-type heating component has a smaller heating area and a smaller contact area with the aerosol generating substrate, and a higher temperature is required to provide sufficient energy to atomize the aerosol generating substrate to generate the aerosol. The other parts are the same as those in the above embodiment and will not be repeated here.

In an embodiment, the processor 110 is further configured to:
after detecting the startup instruction, control the heating component to heat up to the third temperature, in which the third temperature is greater than the first temperature, and control the heating component to decrease from the third temperature to the first temperature.

Specifically, in order to improve the user experience, in the embodiment, after the startup instruction is detected, the heating component in the aerosol generation device is controlled to heat up to the third temperature, in which the third temperature is greater than the first temperature.

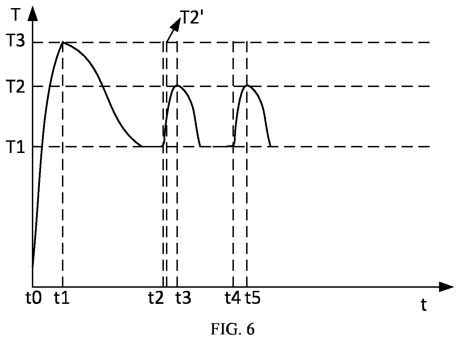

As an example, as shown in FIG. 6, in the embodiment, within a first time period (from t0 to t1), the heating component in the aerosol generation device is controlled to heat up to the third temperature. In the embodiment, the third temperature T3 is greater than the first temperature T1. Since when the aerosol generation device is not started up, the temperatures of the heating component, the aerosol generating substrate and other modules in the aerosol generation device are all lower (generally equal to the ambient temperature of the environment where the aerosol generation device is located, such as 25°C), and there is no aerosol in an atomized state in the aerosol generation device. If the heating component is heated up to the first temperature, there is still no aerosol in the atomized state in the aerosol generation device. In the first part of the period during which the user inhales (such as the time period from t2 to t2' shown in FIG. 6), the user cannot inhale the aerosol, resulting in unsatisfied user experience. Therefore, in the embodiment, after the startup instruction is detected, the heating component is controlled to heat up to the third temperature. In the embodiment, the third temperature is greater than the second temperature, and the second temperature is greater than the first temperature. In such a manner, a small amount of aerosol in the atomized state can be generated in the aerosol generation device for the user to inhale, thereby improving the user experience and allowing the aerosol generating substrate to be fully preheated for subsequent inhalations.

The first temperature is less than or equal to the minimum temperature for generating the aerosol. As an example, the first temperature is between 170°C and 180°C, that is, the first temperature can be any temperature value between 170°C and 180°C. The second temperature is between 210°C and 240°C, that is, the second temperature can be any temperature value between 210°C and 240°C. The third temperature is between 240°C and 260°C, that is, the third temperature can be any temperature value between 240°C and 260°C.

When the heating component reaches the third temperature, the processor controls the energy supplied to the heating component, so that the heating component is cooled down from the third temperature to the first temperature and maintained at the first temperature, thereby reducing the overall power consumption of the aerosol generation device.

During the subsequent use, the detection data of the inhalation detection sensor in the aerosol generation device is acquired, and it is determined whether there exists an inhalation action according to the detection data. The step that the heating component is controlled to heat up or cool down from the current temperature to the second temperature when an inhalation action is detected may include: the heating component is controlled to reach the second temperature from the first temperature, or the heating component is controlled to heat up or cool down from any temperature between T1 and T3 to the second temperature.

In an embodiment, when the aerosol generation device may further include a temperature measuring element, the processor 110 is further configured to: acquire a detected temperature of the heating component through the temperature measuring element in the aerosol generation device; perform a Proportion Integral Differential (PID) calculation according to the detected temperature and a target temperature and obtain target energy, to control the heating component to reach a corresponding target temperature according to the target energy, in which the target temperature includes the first temperature, the second temperature or the third temperature.

In the embodiment, in the process of controlling the temperature of the heating component, the detected temperature of the heating component is acquired by the temperature measuring element in the aerosol generation device, and then the detected temperature and the target temperature serve as PID inputs, and the target energy is obtained after the PID calculation, that is, control information of the heating component is obtained; the heating component is controlled to reach the corresponding target temperature according to the target energy obtained by the calculation, in which the target temperature includes the first temperature, the second temperature or the third temperature.

Specifically, in the startup phase, i.e., after the startup instruction is acquired, the first temperature or the third temperature is the target temperature, and the first temperature or the third temperature and the detected temperature of the heating component acquired by the temperature measuring element serve as the PID inputs to obtain the target energy after the PID calculation. During use, the target temperature can be the first temperature or the second temperature. During the inhalation, the target temperature is the second temperature. After the inhalation, the target temperature is the first temperature. The first temperature or the second temperature and the detected temperature of the heating component acquired by the temperature measuring element serve as the PID inputs and the target energy is obtained after the PID calculation.

After the target energy is obtained, the heating component is controlled to reach the corresponding target temperature according to the target energy. In the embodiment, a deviation of a controlled object (such as the heating component) can be effectively corrected through the PID algorithm, so that the heating component can reach a stable state.

In an embodiment, the processor 110 is further configured to: acquire energy supplied to the heating component; and stop supplying energy to the heating component when the energy supplied to the heating component exceeds preset energy.

Specifically, in the embodiment, during use, the processor may further acquire the energy supplied by the power supply to the heating component in real time. The preset energy can be energy corresponding to the first temperature, the second temperature or the third temperature. As an example, in the startup phase, the preset energy may be energy corresponding to the first temperature or the third temperature. The energy supplied to the heating component by the power supply is detected, and then it is detected that the supplied energy exceeds the preset energy, the energy supply to the heating component is stopped.

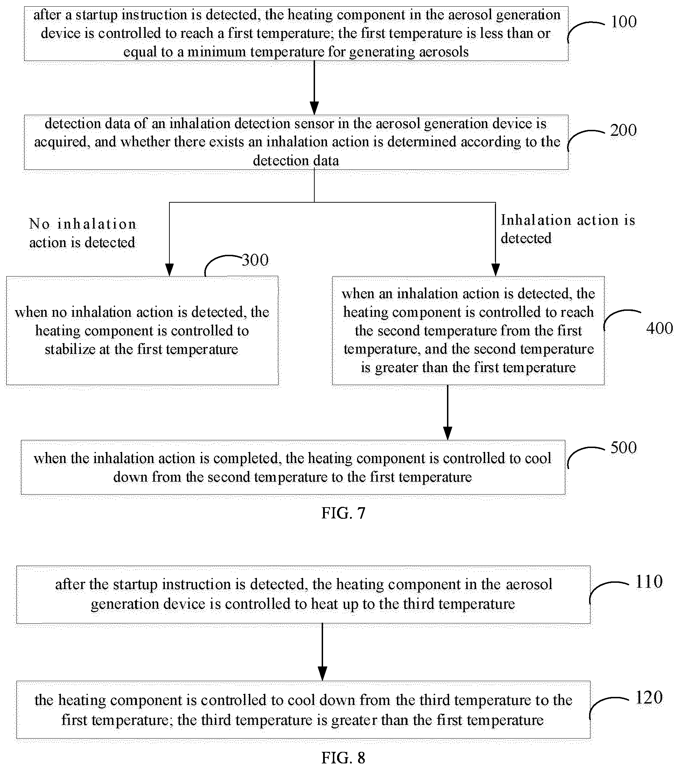

It should be appreciated that the preset energy in the embodiment can be implemented in combination with the embodiment shown in FIG. 7. Specifically, during use, the first temperature, the second temperature or the third temperature, and the current detected temperature serve as the PID inputs, the preset energy is obtained after the PID calculation, and the energy supplied to the heating component is obtained in real time (i.e., the preset energy is obtained). When the energy supplied to the heating component exceeds the preset energy, the energy supply to the heating component is stopped. The adoption of the PID calculation during use can avoid a control error caused by attenuation of each module, and improve the control accuracy.

In an embodiment, after detecting the startup instruction, the aerosol generation device acquires the detected temperature of the heating component through the temperature measuring element, and then take the detected temperature and the first temperature as the PID inputs to perform the PID calculation, and obtain the target energy. The aerosol generation device controls the power supply to supply energy to the heating component according to the target energy, to allow the heating component to reach the first temperature. It takes 5 seconds for the heating component to reach the first temperature.

Subsequently, the detection data of the inhalation detection sensor is acquired, and it is determined whether there exists an inhalation action according to the acquired detection data. When an inhalation action is detected, the target energy is calculated according to the first temperature and the second temperature as the PID inputs, and the heating component is controlled according to the target energy to reach the second temperature from the first temperature, that is, the heating component is controlled to heat to atomize the aerosol generating substrate to form the aerosol. When no inhalation action is detected, the heating component is stabilized at the first temperature to reduce the power consumption in the non-inhalation phase.

In an embodiment, as shown in FIG. 7, a control method for an aerosol generation device is provided, which may specifically include the following steps 100 to 500.

Step 100: after a startup instruction is detected, the heating component in the aerosol generation device is controlled to reach a first temperature; the first temperature is less than or equal to a minimum temperature for generating aerosol.

The present application is applied to an aerosol generation device which may be any aerosol generation device on the market, and is not limited here.

Specifically, the processor in the aerosol generation device can detect whether a startup instruction is received. The startup instruction can be generated by a user operating a power-on instruction on the aerosol generation device. As an example, the aerosol generation device may further include a button for generating a power-on instruction, and the user can press or touch the button to generate a corresponding power-on instruction. Alternatively, there exists an acceleration sensor on the aerosol generation device, and in the user use, the acceleration sensor generates an acceleration, so that the aerosol generation device generates a corresponding startup instruction.

After detecting the startup instruction, the processor in the aerosol generation device controls the heating component in the aerosol generation device to reach the first temperature. The first temperature is less than or equal to the minimum temperature for generating aerosol. Specifically, the processor may control the power supply to provide energy to the heating component to allow the heating component to reach the first temperature.

Furthermore, in order to improve the user experience, the heating component needs to reach the first temperature as quickly as possible to preheat the aerosol generating substrate in the aerosol generation device. The user can inhale the aerosol in less time without having to wait for a long time. In the embodiment, the time required to control the heating component to reach the first temperature is less than 10 seconds.

Step 200: detection data of an inhalation detection sensor in the aerosol generation device is acquired, and whether there exists an inhalation action is determined according to the detection data.

In the embodiment, the aerosol generation device acquires the detection data of the inhalation detection sensor in the aerosol generation device, and determines whether there exists an inhalation action according to the acquired detection data. The detection data may be air pressure change data in the airflow channel, or temperature change data in the airflow channel.

It should be appreciated that the aerosol generation device may also detect the inhalation action according to the temperature change of the heating component detected by the temperature measuring element. In this case, the aerosol generation device may exclude the inhalation detection sensor.

Step 300: when no inhalation action is detected, the heating component is controlled to stabilize at the first temperature.

According to the determination result of step 200, when no inhalation action is detected, the heating component is controlled to stabilize at the first temperature, and the first temperature is less than or equal to the minimum temperature for generating aerosol, as shown in FIG. 2, T1 represents the first temperature.

It should be appreciated that, for ease of use, the first temperature should not be much lower than the minimum temperature for generating aerosol. As an example, when the heating component is a circumferential-shape heating body, the first temperature is less than 200°C, the second temperature is greater than 200°C, and the third temperature is greater than 200°C. Specifically, the first temperature is between 170°C and 180°C, i.e., the first temperature can be any temperature value between 170°C and 180°C. In this case, the minimum temperature for generating aerosol is equal to 180°C. The first temperature should not be set to 100°C, because in the user use, the aerosol generation device may need more time to heat the heating component to the required temperature compared to when the first temperature is equal to 180°C. When the heating component is an inserted-type heating body, the first temperature is less than 300°C, the second temperature is greater than 300°C, and the third temperature is greater than 300°C.

Step 400: when an inhalation action is detected, the heating component is controlled to reach the second temperature from the first temperature, and the second temperature is greater than the first temperature.

According to the determination result of step 200, when an inhalation action is detected, the heating component is controlled to reach the second temperature from the first temperature, that is, the heating component is controlled to heat to atomize the aerosol generating substrate to form aerosol. The second temperature is greater than the first temperature.

Step 500: when the inhalation action is completed, the heating component is controlled to cool down from the second temperature to the first temperature.

After the inhalation action is determined according to the detection data of the inhalation detection sensor, the processor in the aerosol generation device controls the heating component to cool down from the second temperature to the first temperature.

As an example, as shown in FIG. 3, at moment t0, a startup instruction is detected, and the processor in the aerosol generation device controls the heating component in the aerosol generation device to reach the first temperature T1 within time t1. When no inhalation action is detected, the heating component is controlled to stabilize at the first temperature T1. At moment t2, the inhalation action is detected, and the heating component is controlled to increase the temperature from the first temperature T1 to the second temperature T2, to atomize the aerosol generating substrate to form aerosol. At moment t3, it is determined that the inhalation action stops according to the detection data of the inhalation detection sensor, and the processor controls the power supply to reduce the energy supplied to the heating component, so that the temperature of the heating component gradually decreases to the first temperature T1 and is maintained at the first temperature T1. When the next inhalation action is detected, the operations from the moment t2 to moment t3 are repeated, and the cycle is repeated. When a power-off instruction is detected, the power supply stops supplying power to the processor and other modules, the aerosol generation device is in a turn-off state, and the temperature of the heating component decreases to the normal temperature (referring to the ambient temperature of the environment where the aerosol generation device is located).

As an example, the second temperature may be between 210°C and 240°C, i.e., the second temperature may be any temperature value between 210°C and 240°C.

In the above control method for the aerosol generation device, the heating component in the aerosol generation device is controlled to reach the first temperature after a startup instruction is detected, and the first temperature is less than or equal to the minimum temperature for generating aerosol, so that the heating component can preheat the aerosol generating substrate; then the detection data of the inhalation detection sensor in the aerosol generation device is acquired, and it is determined whether there exists an inhalation action according to the detection data; when no inhalation action is detected, the heating component is controlled to stabilize at the first temperature. In such a manner, after the aerosol generation device is turned on, when no inhalation action is detected, the heating component is controlled to stabilize at the first temperature lower than the minimum temperature for generating aerosol. Compared to the conventional technology (continuous heating after being turned on), a lot of power consumption can be saved without affecting the user use.

In an embodiment, as shown in FIG. 8, after the startup instruction is detected, the step of controlling the heating component in the aerosol generation device to reach the first temperature may include:

Step 110: after the startup instruction is detected, the heating component in the aerosol generation device is controlled to heat up to the third temperature.

As an embodiment, in order to improve the user experience, in the embodiment after the startup instruction is detected, the heating component in the aerosol generation device is controlled to heat up to the third temperature within a first time period. The third temperature is greater than the first temperature.

For example, as shown in FIG. 6, in the embodiment the heating component in the aerosol generation device is controlled to heat up to the third temperature within the first time period (from t0 to t1). In the embodiment, the third temperature T3 is greater than the first temperature T1. Since when the aerosol generation device is not started up, the temperatures of the heating component, the aerosol generating substrate and other modules in the aerosol generation device are low (generally the ambient temperature of the environment where the aerosol generation device is located, such as 25°C), and there is no aerosol in the atomized state in the aerosol generation device. When the heating component heats up to the first temperature, there is still no aerosol in the atomized state in the aerosol generation device. In the first part of the period during which the user inhales (such as the time period from t2 to t2' shown in FIG. 6), the user cannot inhale the aerosol, resulting in unsatisfied user experience. Therefore, in the embodiment, after the startup instruction is detected, the heating component is controlled to heat up to the third temperature. In the embodiment, the third temperature is greater than the second temperature, and the second temperature is greater than the first temperature. In such a manner, a small amount of aerosol in the atomized state can be generated in the aerosol generation device for the user to inhale, thereby improving the user experience and allowing the aerosol generating substrate to be fully preheated for subsequent inhalations.

The first temperature is less than or equal to the minimum temperature for generating aerosol. As an example, the first temperature is between 170°C and 180°C, that is, the first temperature may be any temperature value between 170°C and 180°C; the second temperature is between 210°C and 240°C, that is, the second temperature may be any temperature value between 210°C and 240°C; the third temperature is between 240°C and 260°C, that is, the third temperature may be any temperature value between 240°C and 260°C.

Step 120: the heating component is controlled to cool down from the third temperature to the first temperature; the third temperature is greater than the first temperature.

When the heating component heats up to the third temperature, the processor controls the energy supplied to the heating component so that the heating component is cooled down from the third temperature to the first temperature and maintained at the first temperature, thereby reducing the overall power consumption of the aerosol generation device.

During subsequent use, the detection data of the inhalation detection sensor in the aerosol generation device is acquired, and whether there exists an inhalation action is determined according to the detection data. The step that the heating component is controlled to heat up or cool down from the current temperature to the second temperature when an inhalation action is detected may include: the heating component is controlled to reach the second temperature from the first temperature, or the heating component is controlled to heat up or cool down from any temperature between T1 and T3 to the second temperature.

In an embodiment, based on the above embodiment, the method may further include:
the detected temperature of the heating component is acquired through a temperature measuring element in the aerosol generation device;
a PID calculation is performed according to the detected temperature and the target temperature to obtain target energy, to control the heating component to reach a corresponding target temperature according to the target energy; the target temperature includes the first temperature, the second temperature or the third temperature.

In the embodiment, in the process of controlling the temperature of the heating component, the detected temperature of the heating component is acquired by the temperature measuring element in the aerosol generation device, and then the detected temperature and the target temperature serve as proportion integral differential (PID) inputs, the target energy is obtained after the PID calculation, that is, the control information of the heating component is obtained, and the heating component is controlled to reach the corresponding target temperature according to the target energy obtained by the calculation. The target temperature includes the first temperature, the second temperature or the third temperature.

Specifically, in the startup phase, that is, after the startup instruction is acquired, the first temperature or the third temperature is the target temperature, and the first temperature or the third temperature and the detected temperature of the heating component acquired by the temperature measuring element serve as the PID inputs, and the target energy is obtained through the PID calculation. During use, the target temperature may be the first temperature or the second temperature. During inhalation, the target temperature is the second temperature. After inhalation, the target temperature is the first temperature. The first temperature or the second temperature and the detected temperature of the heating component obtained by the temperature measuring element serve as the PID inputs and the target energy is obtained after the PID calculation.

After the target energy is acquired, the heating component is controlled to reach the corresponding target temperature according to the target energy. In the embodiment, the deviation of the controlled object (i.e., the heating component) can be effectively corrected through the PID algorithm, so that the heating component reaches a stable state.

In an embodiment, based on the above embodiment, the method may further include:
the energy supplied to the heating component is acquired;
when the energy supplied to the heating component exceeds preset energy, the energy supply to the heating component is stopped.

Specifically, in the embodiment, during use, the processor may further acquire the energy supplied by the power supply to the heating component in real time. The preset energy may be energy corresponding to the first temperature, the second temperature or the third temperature. For example, in the startup phase, the preset energy can be the energy corresponding to the first temperature or the third temperature. The energy supplied to the heating component by the power supply is detected. When it is detected that the supplied energy exceeds the preset energy, the energy supply to the heating component is stopped.

It should be appreciated that the preset energy in the embodiment can be implemented in combination with the embodiment shown in FIG. 7. Specifically, during use, the first temperature, the second temperature or the third temperature, and the currently detected temperature serve as the PID inputs, the preset energy is obtained through the PID calculation, and the energy supplied to the heating component is acquired in real time (i.e., the preset energy is obtained). After the energy supplied to the heating component exceeds the preset energy, the energy supply to the heating component is stopped. The adoption of the PID calculation during use can avoid a control error caused by attenuation of each module and improve the control accuracy.

In an embodiment, after detecting the startup instruction, the aerosol generation device acquires the detected temperature of the heating component through the temperature measuring element, and then takes the detected temperature and the first temperature as the PID inputs to perform the PID calculation and obtain the target energy. The aerosol generation device controls the power supply to supply energy to the heating component according to the target energy, to allow the heating component to reach the first temperature. It takes 5 seconds for the heating component to reach the first temperature.

The detection data of the inhalation detection sensor is acquired, whether there exists an inhalation action is determined according to the acquired detection data. When an inhalation action is detected, the target energy is obtained through the calculation according to the first temperature and the second temperature as the PID inputs, and the heating component is controlled to reach the second temperature from the first temperature according to the target energy, that is, the heating component is controlled to heat to atomize the aerosol generating substrate to form aerosol. When no inhalation action is detected, the heating component is stabilized at the first temperature to reduce power consumption in the non-inhalation phase.

It should be appreciated that, although the steps in the flow charts involved in the embodiments described above are displayed sequentially as indicated by the arrows, these steps are not definitely executed sequentially in the order indicated by the arrows. Unless otherwise specified herein, there is no strict order limitation for the execution of these steps, and these steps may be executed in other orders. Moreover, at least a part of the steps in the flow charts involved in the embodiments described above may include multiple steps or multiple phases. These steps or phases are not definitely executed at the same time, but can be executed at different moments. These steps or phases are not definitely executed sequential, but can be executed in turn or alternately with other steps or at least part of the steps or phases in other steps.

Based on the same inventive concept, in an embodiment of the present invention, a control apparatus for implementing the above-mentioned control method for the aerosol generation device is provided. The solution to the problem provided by the apparatus is similar to the solution described in the above method. Therefore, for the specific limitations in one or more embodiments of the aerosol generation apparatus provided below, reference can be made to the limitations on the control method for the aerosol generation device described above, which will not be repeated here.

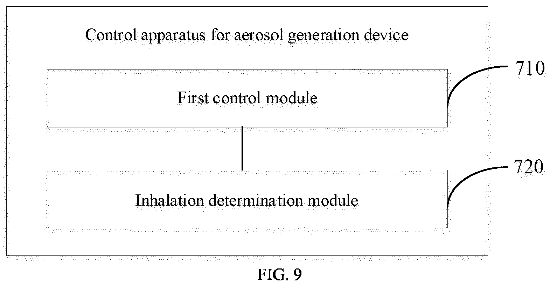

In an embodiment, as shown in FIG. 9, a control apparatus for an aerosol generation device is provided, including:
a first control module 710, configured to control a heating component in the aerosol generation device to reach a first temperature after a startup instruction is detected, in which the first temperature is less than or equal to a minimum temperature for generating aerosol;
an inhalation determination module 720, configured to acquire detection data of an inhalation detection sensor in the aerosol generation device, and determine whether there exists an inhalation action according to the detection data.

The first control module 710 is further configured to control the heating component to stabilize at the first temperature when no inhalation action is detected.

In an embodiment, the control apparatus for the aerosol generation device may further include:
a second control module 730, configured to control the heating component to reach a second temperature from the first temperature when an inhalation action is detected, in which the second temperature is greater than the first temperature.

In an embodiment, the control apparatus for the aerosol generation device may further include:
a third control module 740, configured to control the heating component to cool down from the second temperature to the first temperature after the inhalation action is completed.

In an embodiment, the first control module 710 is further configured to control the heating component in the aerosol generation device to heat up to a third temperature after a startup instruction is detected, and control the heating component to cool down from the third temperature to the first temperature, in which the third temperature is greater than the first temperature.

In an embodiment, the heating component is a circumferential-shape heating body, the first temperature is less than 200°C, the second temperature is greater than 200°C, and the third temperature is greater than 200°C.

In an embodiment, the first temperature is between 170°C and 180°C, the second temperature is between 210°C and 240°C, and the third temperature is between 240°C and 260°C.

In an embodiment, the heating component is an inserted-type heating body, the first temperature is less than 300°C, the second temperature is greater than 300°C, and the third temperature is greater than 300°C.

In an embodiment, the control apparatus for the aerosol generation device may further include:
a first acquisition module (not shown), configured to acquire a detected temperature of the heating component through a temperature measuring element in the aerosol generation device;
a calculation module (not shown), configured to perform a PID calculation according to the detected temperature and a target temperature and obtain target energy, to control the heating component to reach a corresponding target temperature according to the target energy, in which the target temperature includes the first temperature, the second temperature or the third temperature.

In an embodiment, the control apparatus for the aerosol generation device may further include:
a second acquisition module (not shown), configured to acquire energy supplied to the heating component;
a fourth control module (not shown), configured to stop supplying energy to the heating component when the energy supplied to the heating component exceeds preset energy.

The modules in the above control apparatus can be fully or partially implemented by software, hardware or a combination thereof. The above modules may be embedded in or independent of a processor in a computer device in the form of hardware, or may be stored in a memory in a computer device in the form of software, so that the processor can invoke and execute operations corresponding to the above modules.

In an embodiment, a computer-readable storage medium is provided, on which a computer program is stored. The computer program, when executed by a processor, may cause the processor to implement the steps in any of the above-mentioned embodiments of the control method for the aerosol generation device.

A person of ordinary skill in the art can understand that all or part of the processes in the above-mentioned embodiments of the method can be implemented by instructing related hardware through a computer program. The computer program may be stored in a non-transitory computer-readable storage medium. When the computer program is executed, the processes of the embodiments of the above-mentioned method are included. Any reference to a memory, a database, or other medium used in the embodiments provided in the present application may include at least one of a non-transitory memory and a transitory memory. The non-transitory memory may include a read-only memory (ROM), a magnetic tape, floppy disk, a flash memory, an optical storage, a high-density embedded non-transitory memory, a resistive random access memory (ReRAM), a magnetoresistive random access memory (MRAM), a ferroelectric random access memory (FRAM), a phase change memory (PCM), a graphene memory, etc. The transitory memory may include a random access memory (RAM) or an external cache memory, etc. By way of illustration and not limitation, the RAM may be in various forms, such as a static random access memory (SRAM) or a dynamic random access memory (DRAM). The database involved in each embodiment of the present invention may include at least one of a relational database and a non-relational database. The non-relational database may include, but is not limited to, a distributed database based on blockchain. The processor involved in each embodiment of the present invention may be a general-purpose processor, a central processing unit, a graphics processor, a digital signal processor, a programmable logic unit, a data processing logic unit based on quantum computing, etc., but is not limited thereto.

The technical features in the above embodiments may be combined arbitrarily. In order to make the description concise, all possible combinations of the technical features in the above embodiments are not described. However, as long as there is no contradiction in the combinations of these technical features, these combinations should be considered to be within the scope of the present application.

The above-described embodiments only express several implementation modes of the present invention, and the descriptions are relatively specific and detailed, but should not be construed as limiting the scope of the present invention. It should be noted that, those of ordinary skill in the art can make several modifications and improvements without departing from the concept of the present invention, and these all fall within the protection scope of the present invention. Therefore, the protection scope of the present invention should be subject to the appended claims.

## Claims

1. An aerosol generation device, comprising a power supply, a processor, a heating component, and an inhalation detection sensor; wherein
the power source is configured to supply energy to the heating component;
the processor is configured to control the energy supplied to the heating component after detecting a startup instruction, to allow the heating component to reach a first temperature which is less than or equal to a minimum temperature for generating an aerosol;
the processor is further configured to acquire detection data of the inhalation detection sensor, determine whether there exists an inhalation action according to the detection data, and control the heating component to stabilize at the first temperature when no inhalation action is detected.

2. The aerosol generation device according to claim 1, wherein the processor is further configured to control the heating component to reach a second temperature from the first temperature when the inhalation action is detected, wherein the second temperature is greater than the first temperature.

3. The aerosol generation device according to claim 2, wherein the processor is further configured to control the heating component to cool down from the second temperature to the first temperature after the inhalation action is completed.

4. The aerosol generation device according to claim 1, wherein the processor is further configured to:
control the heating component to heat up to a third temperature after detecting the startup instruction, the third temperature being greater than the first temperature; and
control the heating component to cool down from the third temperature to the first temperature.

5. The aerosol generation device according to claim 4, wherein the heating component is configured to receive an aerosol generating substrate and heat around the aerosol generating substrate, the first temperature is less than 200°C, the second temperature is greater than 200°C, and the third temperature is greater than 200°C.

6. The aerosol generation device according to claim 5, wherein the first temperature is between 170°C and 180°C, the second temperature is between 210°C and 240°C, and the third temperature is between 240°C and 260°C.

7. The aerosol generation device according to claim 4, wherein the heating component is configured to be inserted into the aerosol generating substrate and heat the aerosol generating substrate from an inside of the aerosol generating substrate, the first temperature is less than 300°C, the second temperature is greater than 300°C, and the third temperature is greater than 300°C.

8. The aerosol generation device according to any one of claims 1 to 7, further comprising a temperature measuring element, wherein the processor is further configured to:
acquire a detected temperature of the heating component through the temperature measuring element;
perform a PID calculation according to the detected temperature and a target temperature to obtain a target energy, and control the heating component to reach a corresponding target temperature according to the target energy, wherein the target temperature comprises the first temperature, the second temperature or the third temperature.

9. The aerosol generation device according to claim 8, wherein the processor is further configured to:
acquire an energy supplied to the heating component, and stop supplying energy to the heating component when the energy supplied to the heating component exceeds preset energy.

10. A control method for an aerosol generation device, comprising:
controlling a heating component in the aerosol generation device to reach a first temperature after a startup instruction is detected, the first temperature being less than or equal to a minimum temperature for generating an aerosol;
acquiring detection data of an inhalation detection sensor in the aerosol generation device, and determining whether there exists an inhalation action according to the detection data;
controlling the heating component to stabilize at the first temperature when no inhalation action is detected.

11. The method according to claim 10, further comprising:
after acquiring the detection data of the inhalation detection sensor in the aerosol generation device and determining whether there exists the inhalation action according to the detection data,
controlling the heating component to reach the second temperature from the first temperature when the inhalation action is detected, the second temperature being greater than the first temperature.

12. The method according to claim 11, further comprising:
after controlling the heating component to reach the second temperature from the first temperature when the inhalation action is detected,
controlling the heating component to cool down from the second temperature to the first temperature when the inhalation action is completed.

13. The method according to claim 10, further comprising:
after controlling the heating component in the aerosol generation device to reach the first temperature after the startup instruction is detected,
controlling the heating component in the aerosol generation device to heat up to the third temperature after the startup instruction is detected; and
controlling the heating component to cool down from the third temperature to the first temperature.

14. A control apparatus for an aerosol generation device, comprising:
a first control module, configured to control a heating component in the aerosol generation device to reach a first temperature after a startup instruction is detected, wherein the first temperature is less than or equal to a minimum temperature for generating an aerosol;
an inhalation determination module, configured to acquire detection data of an inhalation detection sensor in the aerosol generation device, and determine whether there exists an inhalation action according to the detection data;
wherein the first control module is further configured to control the heating component to stabilize at the first temperature when no inhalation action is detected.
